# EUROPEAN PATENT APPLICATION

(11) **EP 2 987 525 A1**
(43) Date of publication of application: **24.02.2016**
(21) Application number: 14728310.5
(22) Date of filing: 17.04.2014
(51) Int. Cl.: A61M 16/10, A61M 16/22, B01D 53/22

(54) **PROCESS FOR THE PURIFICATION OF ANESTHESIA GASES USING MEMBRANE CONTACTORS AND ITS APPLICATIONS**

(30) Priority: 19.04.2013 PT 10690213
(71) Applicant: Graça Neves, Luísa Alexandra, 2820-142 Charneca Da Caparica (PT); Rôla Coelhoso, Isabel Maria, 2815-694 Sobreda (PT); Mateus Afonso, Carlos Alberto, 2815-752 (PT); Serejo Goulão Crespo, João Paulo, 1300-319 Lisboa (PT)
(72) Inventor: Graça Neves, Luísa Alexandra, 2820-142 Charneca Da Caparica (PT); Rôla Coelhoso, Isabel Maria, 2815-694 Sobreda (PT); Mateus Afonso, Carlos Alberto, 2815-752 (PT); Serejo Goulão Crespo, João Paulo, 1300-319 Lisboa (PT)
(74) Representative: Ferreira, Maria Silvina
(86) International application number: PCT/IB2014/060797
(87) International publication number: WO 2014/170858

(57) **Abstract**

The present application describes a process for removing toxic compounds, namely carbon dioxide, present in gas streams in anesthesia circuits, using membrane contactors. The present process allows for the recirculation of anesthesia gases, CO₂ free, to the anesthetized subject, as well as the recirculation of the receiving absorber liquid phase, from which the CO₂ is removed.

The present application also describes the use of this procedure in the field of medicine, namely its *in-situ* use during medical interventions, in which the subject is anesthetized.

## Description

### Technical domain

The present application refers to a process for the removal of toxic compounds, namely carbon dioxide present in gas streams in anesthesia circuits.

### Background

An anesthesia gas is usually composed of about 70% of nitrous oxide (N₂O), 22-29% of oxygen and 1-8% of halogenated hydrocarbons. After respiration, the carbon dioxide (CO₂) present in the gas mixture reaches a content of 5% and must be removed to values lower than 0.5%, making it possible to recirculate the gas to the patient under anesthesia.

Currently, the removal of CO₂ from anesthesia gas streams is made by using an absorbent comprising calcium hydroxide (CaOH₂). The absorbent promotes the following reaction with the CO₂:

CaOH₂ (s) + CO₂ (g) → Ca (CO₃) (s) + H₂O (g, 1)

in which the calcium hydroxide reacts with the carbon dioxide to form calcium carbonate and water. Although being the absorbent most used in anesthesia circuits for CO₂ removal, calcium hydroxide has high sensitivity to reactive anesthetic agents. Anesthetic agents most commonly used are fluorinated hydrocarbons, such as sevoflurane, isoflurane, desflurane, which can chemically react with calcium hydroxide to form potentially toxic by-products to the patient.

Some solutions are proposed in the literature to minimize the occurrence of secondary reactions between the absorbent and the anesthetic agent. Patent MX 2009006916A suggests the use of a CO₂ absorbent having the composition of 70-90% calcium hydroxide (potassium hydroxide and sodium hydroxide free), 0.1 to 17% of lithium hydroxide and 5-25% of water. U.S. Patent 5,390,667 suggests the use of a CO₂ absorbent comprising magnesium, magnesium hydroxide or magnesium hydrocarbon with a small amount of water.

All the solutions mentioned above are based on absorption systems, meaning that no simultaneous regeneration of the absorbent occurs, which implies the replacement of the absorbent when it is saturated and high cost associated with the disposal of contaminated hospital waste.

More recently, U.S. 2004/0103898 suggests the construction of a pipe to adjust to anesthesia circuits, which should contain a membrane and a CO₂ absorbent (without specifying the composition neither of the absorbent nor of the membrane material) being however, once again, only one absorption system, which does not ensure its regeneration *in situ*.

As mentioned above, the most commonly used anesthetic agents are fluorinated hydrocarbons that may lead to the formation of potentially toxic compounds when combined with the absorbent.

Xenon, on the other hand, is considered an ideal anesthetic gas. Its use makes practically no side effects to occur (particularly in the cardiovascular system), is odorless, and allows rapid induction.

In addition, it does not react chemically with any substance, not presenting the problem of formation of potentially toxic compounds to the patient. However, the high cost of surgery when using Xenon compared with other anesthetic agents has limited its use. Thus it is urgent to find efficient alternative processes to recover and recycle anesthesia gases, particularly xenon, efficiently and cost effectively.

Recent works propose the use of membranes for CO₂ absorption, using solutions of salts of amino acids with polyethylene glycol and glycerol, but these processes require thermal regeneration, which limits its use in hospital environment (Portugal, AF, FD Magalhaes, A. Mendes, Carbon dioxide removal from anesthetic gas circuits using hollow fiber membrane contactors with amino acid salt solutions J. Memb Sci. 339 (2009) 275-286).

Some ionic liquids (ILs) present high solubility to CO₂, which makes them suitable candidates as CO₂ absorbents in gas streams, in addition of having a very low vapor pressure, which ensures no losses to the atmosphere, by evaporation.

Also there are eutectic mixtures which provide a high affinity for CO₂ and negligible volatility.

The present invention provides an integrated process (*in-situ* absorption and regeneration) for the purification of anesthesia gases and CO₂ removal combining the ability of an ionic liquid or eutectic mixtures with specificity for solubilizing carbon dioxide using an enzyme, carbonic anhydrase, which catalyzes the conversion reaction of the CO₂ to bicarbonate according to the following reaction:

CO₂ + H₂O ⇄ HCO₃⁻ + H⁺

Thus, in addition to the CO₂ transport by the selective liquid phase, the additional mechanism of enzymatic conversion in bicarbonate increases the driving force for transport, greatly improving the total throughput of CO₂. Carbonic anhydrase is recognized by the scientific community as having the ability to reversibly catalyze the conversion reaction of carbon dioxide in bicarbonate

### Summary

It is a purpose of this application the description of a purification process of gases from anesthesia circuits using membrane contactors. This process is characterized by contacting a gaseous stream comprising anesthesia gases and exhaled gases by the anesthetized subject, in a membrane contactor, ensuring the solubility and diffusion of toxic gaseous compounds in a receiving liquid phase which integrates the carbonic anhydrase enzyme dispersed in an ionic liquid or eutectic mixture with affinity to the carbon dioxide, allowing its subsequent removal from this liquid phase, in a second membrane contactor.

Thus, said process allows the recirculation of the purified anesthesia gas stream to the subject under intervention and the recirculation of the receiving liquid phase, CO₂ free (at the exit of the second contactor), to the first contactor.

The main advantages are:
a. Elimination of toxicity problems caused by the current gas purification systems in anesthesia circuits.
b. *In situ* regeneration and at room temperature of the liquid phase used for solubilisation of toxic gases.
c. Compact equipment (membrane contactors) suitable for use in hospital environment.

In this context, the present application describes a process for removing the carbon dioxide present in the gas stream in anesthesia circuits, comprising the steps of:
- admission of a gaseous stream comprising anesthesia and exhaled gases by the subject, to at least a first membrane contactor, in which occurs the solubilisation and diffusion of the carbon dioxide to a receiving liquid phase;
- admission of the receiving liquid phase from the first contactor, to at least one second membrane contactor in which the stripping of the carbon dioxide occurs;
- recirculation of the gas stream from the first contactor to the anesthesia gas stream to be administered to the subject;
- recirculation of the liquid phase from the second contactor to the first contactor.

In a preferred embodiment, the receiving liquid phase comprises an ionic liquid or a eutectic mixture.

In another preferred embodiment, the receiving liquid phase further comprises an immobilized carbonic anhydrase enzyme, with concentrations ranging from 0.001% (w/w) to 0.1% (w/w).

In yet another preferred embodiment, the ionic liquid presents a Q⁺X⁻ structure in which the cationic moiety Q⁺ is selected from the group comprising imidazolium, pyrazolium, pyridinium, pyrrolidinium, ephedrinium, morpholinium, piperazinum, triazolium, thiazolium, benzotriazole, oxazolium, tetrazolium, sulfonium, ammonium, phosphonium or guanidinium or containing a pending tertiary amine group and wherein the anionic moiety X⁻ is selected from the group comprising halide tetrachloride, aluminum tetrachloride, iron perchlorate, nitrate, cyanide, dicyanamide, thiocyanate, tricianometanide, formate, acetate, trifluoroacetate, trichloroacetate, propionate, butanoate, octanoate, methylsulfate, ethylsulfate, propylsulphate, butylsulfate, octylsulfate, tosylate, benzenesulfonate, mesylate, triflate, sulfate, hydrogen sulfate, carbonate, bis (trifluoromethyl sulfonyl)imide, carborane, saccharine, acesulphanate, hexafluorophosphate, phosphate, dihydrogen phosphate, hydrogen phosphate, tetrafluoroborate, tetraphenylborate.

In a preferred embodiment, the eutectic mixture comprises a combination of choline with a compound selected from the group comprising urea, thiourea, methylurea, 1,3-dimethylurea, 1,1-dimethylurea, acetamide, benzamide, ethylene glycol, glycerol, 2,2,2-trifluoroacetamide, imidazole, adipic acid, benzoic acid, citric acid, malonic acid, oxalic acid, phenylacetic acid, phenylpropionic acid, succinic acid, levulinic acid, xylitol, sorbitol, tartaric acid or isosorbide or a combination of methylurea urea, 1,3-dimethylurea or 1,1-dimethylurea, and choline, triethyl chloroethylammonium chloride, dimethyl benzyl hydroxyethylammonium chloride, triphenyl methylphosphonium chloride and zinc dichloride.

In another preferred embodiment the operating temperature of the receiving liquid phase ranges between 10°C and 100°C, preferably lying at room temperature.

In yet another preferred embodiment, the two contactors are membrane contactors whose pore diameter are between 20nm and 500nm.

In a preferred embodiment the membranes of the two contactors are polymeric or inorganic or simultaneously comprising polymeric materials and inorganic materials, being tubular modules, capillary fibers, hollow fibers, flat modules or spiral modules.

It is also purpose of this application the use of this process in medicine, preferably in medical interventions in which the subject is anesthetized.

### General description

The present application proposes an integrated process (simultaneous absorption and regeneration) using membrane contactors, in order to obtain a compact medical system which allows the recycling of anesthetic gases to the patient during the medical intervention procedure.

In this configuration, the gas stream containing CO₂ contacts with a solution comprising ionic liquids or eutectic mixtures with an adequate water activity and the carbonic anhydrase enzyme. CO₂ is selectively transported from the anesthesia gas stream through the solution comprising ionic liquids or eutectic mixtures containing the carbonic anhydrase enzyme.

This solution is regenerated at room temperature which results in no accumulation of CO₂ in the absorbing solution, thus avoiding its saturation (as in the previously proposed solutions). Elevated temperatures are not necessary to regenerate the ionic liquid or the eutectic mixture, because the reduced pressure or the carrier gas used in the permeate circuit allows the release of the captured carbon dioxide. On the other hand, the treated gas stream with a suitable amount of carbon dioxide can be recirculated to the patient. This system completely avoids contamination of the treated gas because the ionic liquid or the eutectic mixture has a negligible vapor pressure. It has been recently demonstrated in the literature the ability of the ionic liquid ([C₄MIM] [Tf₂N]) to solubilize the carbonic anhydrase enzyme, the increased solubility coefficient of CO₂ being observed in 30% to a small concentration of enzyme (0.1 mg / g ionic liquid) (Neves, Luisa C. Afonso, IM Coelhoso, J. Crespo, Integrated CO2 capture and enzymatic bioconversion in supported ionic liquid membranes, Sep. Sci. Purif. Tech. (2012), 97, 34-41).

In this process, it is proposed the dispersion of the enzyme, carbonic anhydrase in ionic liquids or eutectic mixtures with high affinity to selectively remove CO₂. By adjusting the suitable activity in water it is possible for the carbonic anhydrase (CA) enzyme to be dispersed in an ionic liquid or eutectic mixture. The ionic liquids, compounds consisting entirely of ionic species, possess a Q⁺X⁻ general structure, chiral or achiral, being Q⁺ an organic cation and X⁻ an inorganic or an organic anion. The cationic unit Q⁺ is a unit preferentially of the type imidazolium, pyrazolium, pyridinium, pyrrolidinium, ephedrinium, morpholinium, piperazinum, triazolium, thiazolium, benzotriazole, oxazolium, tetrazolium, sulfonium, ammonium, phosphonium or guanidium and the anionic moiety X⁻ is an halide, aluminum tetrachloride, iron tetrachloride, perchlorate, nitrate, cyanide, dicyanamide, thiocyanate, tricianometanide, formate, acetate, trifluoroacetate, trichloroacetate, propionate, butanoate, octanoate, methylsulfate, ethylsulfate, propylsulphate, butylsulfate, octylsulfate, tosylate, benzenesulfonate, mesylate, triflate, sulfate, hydrogen sulfate, carbonate, bis(trifluoromethyl-sulfonyl)imide, carborane, saccharinate, acesulphanate, hexafluorophosphate, phosphate, dihydrogenophosphate, hydrogenphosphate, tetrafluoroborate, tetraphenylborate.

By presenting a very low vapor pressure they may be used either in supported liquid membranes or in membrane contactors, yielding highly stable membranes or solutions when in contact with gas streams. The use of ionic liquids or eutectic mixtures with very low pressure vapor, with the particularity of concentrating and solubilizing CO₂, promote the perfect environment for the enzyme, ensuring a high local concentration of CO₂ and the required level of water to ensure an adequate activity and stability of the enzyme. Thus, in addition to a greater solubility of CO₂ in a IL or selective eutectic mixture, the additional mechanism of the enzymatic conversion of CO₂ in bicarbonate increases the driving force for transport and significantly improves the overall mass transfer of CO₂. This concept also allows overcoming the problems of loss of absorbent by evaporation which occur when using aqueous solutions. The use of a membrane contactor characterized by a high mass transfer area in a small volume allows the development of a compact equipment, suitable for hospital use and allows recirculation of the anesthesia gases to the patient.

In this process ionic liquids or eutectic mixtures with high affinity for CO₂ are preferably used. In recent experiments it was developed a range of ionic liquids based on the imidazolium cation having high affinity for CO₂. The eutectic mixture comprises a combination of choline and one or more compounds selected from the group consisting of urea, thiourea, methylurea urea, 1,3-dimethylurea, 1,1-dimethylurea, acetamide, benzamide, ethylene glycol, glycerol, 2,2,2-trifluoroacetamide, imidazole, adipic acid, benzoic acid, citric acid, malonic acid, oxalic acid, phenylacetic acid, phenylpropionic acid, succinic acid, levulinic acid, xylitol, sorbitol, tartaric acid or isosorbide or a combination of urea, methylurea, 1,3 - dimethylurea or 1,1-dimethylurea, and choline, triethyl chloroethyl ammonium chloride, dimethyl benzyl-hydroxyethyamonium chloride, triphenylmethylphosphonium chloride, and zinc dichloride. This affinity may be further improved by the synthesis of ionic liquids based on imidazolium cation, pyrazolium, pyridinium, pyrrolidinium, ephedrinium, morpholinium, piperazinum, triazolium, thiazolium, benzotriazole, oxazolium, tetrazolium, sulfonium, ammonium, phosphonium or guanidium, containing a pending tertiary amine group. The use of these cations allows for the selective absorption of CO₂ due to the presence of the organic cation and ensures that the group containing the tertiary amine acts as a counter ion of the bicarbonate anion enzymatically produced.

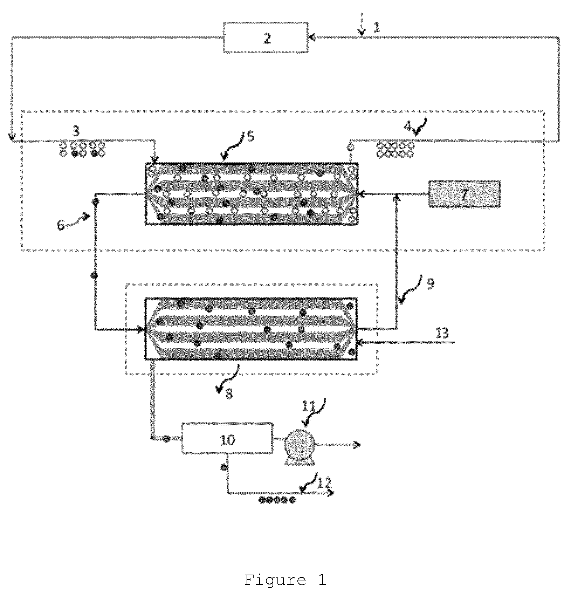

This technology allows solving the problem of removing toxic compounds present in the anesthesia circuit, preferentially CO₂, in an efficient and sustainable way, being the ionic liquid or the eutectic mixture regenerated by the CO₂ stripping using low pressure or a sweep gas (Figure 1).

### Brief Description of the Figures

For an easier understanding of the invention the attached figure is joined, which represents preferred embodiments of the invention that, however, do not intend to limit the object of the present application.

Figure 1. Integrated process for capturing toxic gases present in the anesthesia circuits with two membrane contactors in series. In the first contactor, the CO₂ present in the anesthesia gas is preferably removed and in the second contactor the receiving liquid phase is regenerated in the circuit, with a sweep gas or with reduced pressure.

Reference numbers are:
1 - admission stream of anesthesia gas;
2 - subject or patient;
3 - anesthesia and exhaled gases by the anesthetized subject, rich in CO₂, N₂O and Xe;
4 - gaseous stream of gases, CO₂ free;
5 - first contactor;
6 - stream of the receiving liquid phase with dissolved CO₂;
7 - receiving liquid phase;
8 - second contactor;
9 - CO₂ free receiving liquid phase stream;
10 - condenser;
11 - vacuum pump;
12 - CO₂ recovery;
13 - stream with sweep gas.

### Description of the embodiments

Referring to the figures, some embodiments will now be described in more detail, which however are not intended to limit the scope of the present application.

The present application describes a process for the efficient removal of toxic compounds, namely carbon dioxide, present in the gas stream in anesthesia circuits, combining the capacity of ionic liquids or eutectic mixtures with specificity for solubilizing carbon dioxide using an enzyme, carbonic anhydrase that catalyzes the conversion reaction of CO₂ into bicarbonate.

In this process, the carbonic anhydrase enzyme is dissolved in an ionic liquid or eutectic mixture that presents high solubility to CO₂. In order to ensure the stability and the enzyme activity, it is necessary that this liquid or mixture contains a certain activity in water, with optimum values between 0.2 and 0.8.

In this process, a membrane contactor characterized by a high mass transfer area in a small volume is used. In the integrated process it is foreseen to use two contactors (5 and 8), with membranes that can be polymeric or ceramic, with pore sizes that can range between 0.02 micrometers and 0.5 microns. This process is characterized by contacting a gaseous stream, constituted by anesthesia gases, and by exhaled gases by the anesthetized rich in CO₂, N₂O or Xe (3), in a membrane contactor (5) ensuring the solubility and diffusion of toxic gaseous compounds to a receiving liquid phase (7) that integrates the carbonic anhydrase enzyme dispersed in an ionic liquid or in a eutectic mixture, with affinity for CO₂.

The liquid phase in which CO₂ is dissolved (6) is fed to a second membrane contactor (8) operated under reduced pressure using a condenser (10) and a vacuum pump (11), or using a sweep gas with controlled flow (13). In this second contactor (8), the CO₂ is captured (12) and separated from the stream of the receiving liquid phase which contains the enzyme and the ionic liquid or eutectic mixture, CO₂ free (9), the latter being recirculated to the first contactor (5). The treated gas stream in the first contactor, free of carbon dioxide (4) can be recirculated to the patient (2). It is also possible a controlled addition of the anesthesia gas (1) in order to restore the composition of the anesthesia gas administered to the patient. This system completely avoids the contamination of the treated gas (and of the stream of carbon dioxide) because the ionic liquid or eutectic mixture has negligible vapor pressure. No high temperatures are needed to regenerate the ionic liquid or eutectic mixture, because the use of reduced pressure or the use of a carrier gas in the permeated circuit allows the release of the captured carbon dioxide.

### Example 1

In the ionic liquid 1-butyl-3-methylimidazolium bis (trifluoromethanosulfonil) imide, [C₄MIM] [Tf₂N] with water activity equal to 0.753, the lyophilized carbonic anhydrase enzyme was dissolved, in the concentration of 0.01% (w/w). The liquid phase, consisting of ionic liquid and enzyme was immobilized on a PVDF hydrophobic membrane, with pore size equal to 0.22 micrometers. Permeability tests were performed to the pure gases, carbon dioxide and xenon, at 30°C, to the immobilized hydrophobic membrane with ionic liquid, and to the immobilized hydrophobic membrane with ionic liquid and enzyme. The experimental results showed an average increase of 90% in the ideal selectivity CO₂/Xe when the enzyme is present in the immobilized liquid phase in the pores of the membrane.

## Claims

1. A process for removing carbon dioxide present in the gas stream in anesthesia circuits, comprising the following steps:
- admission of a gaseous stream, comprising anesthesia gases and exhaled gases by the anesthetized subject, to at least a first membrane contactor in which the solubilisation and diffusion of the carbon dioxide to a receiving liquid phase occurs;
- admission of the receiving liquid phase from the first contactor, to the at least one second membrane contactor in which the carbon dioxide stripping occurs;
- recirculation of the gas stream from the first contactor to the gas stream of anesthesia to be administered to the subject;
- recirculation of the receiving liquid phase from the second contactor to the first contactor.

2. The process according to the preceding claim, wherein the receiving liquid phase comprises an ionic liquid or a eutectic mixture.

3. The process according to the preceding claim, wherein the receiving liquid phase further comprises an immobilized carbonic anhydrase enzyme.

4. The process according to the preceding claim, wherein the concentration of the carbonic anhydrase enzyme immobilized in the receiving liquid phase ranges from 0.001% (w/w) to 0.1% (w/w).

5. Process according to any one of the claims 2-4, wherein the ionic liquid has a Q⁺X⁻ structure, wherein the cationic moiety Q⁺ is selected from the group comprising imidazolium, pyrazolium, pyridinium, pyrrolidinium, ephedrinium, morpholinium, piperazinum, triazolium, thiazolium, benzotriazole, oxazolium, tetrazolium, sulfonium, ammonium, phosphonium or guanidinium, or containing a pending tertiary amine group and wherein anionic moiety X⁻ is selected from the group comprising halide, tetrachloride aluminum, iron tetrachloride, perchlorate, nitrate, cyanide, dicyanamide, thiocyanate, tricianometanide, formate, acetate, trifluoroacetate, trichloroacetate, propionate, butanoate, octanoate, methylsulfate, ethylsulfate, propylsulphate, butylsulfate, octylsulfate, tosylate, benzenesulfonate, mesylate, triflate, sulfate, hydrogensulfate, carbonate, bis (trifluoromethyl-sulfonyl)imide, carborane, saccharinate, acesulphanate, hexafluorophosphate, phosphate, dihydrogenphosphate, hydrogenphosphate, tetrafluoroborate , tetraphenylborate.

6. The process according to any one of claims 2-4, wherein the eutectic mixture comprises a combination of choline with a compound selected from the group comprising urea, thiourea, methylurea, 1,3-dimethylurea, 1,1-dimethylurea, acetamide, benzamide, ethylene glycol, glycerol, 2,2,2-trifluoroacetamide, imidazole, adipic acid, benzoic acid, citric acid, malonic acid, oxalic acid, phenylacetic acid, phenylpropionic acid, succinic acid, levulinic acid, xylitol, sorbitol, tartaric acid or isosorbide or by the combination of methylurea urea, 1,3-dimethylurea and 1,1-dimethylurea, and choline, triethyl chloroethylammonium chloride, dimethyl-benzylhydroxyethylammonium chloride, triphenyl methylphosphonium chloride and zinc dichloride.

7. The process according to any one of the preceding claims, wherein the operating temperature of the receiving liquid phase ranges between 10°C and 100°C.

8. The process according to any one of the preceding claims, wherein the operating temperature of the receiving liquid phase is at room temperature.

9. The process according to any preceding claims, wherein the two contactors are membrane contactors whose pore diameter is between 20nm and 500nm

10. The process according to any one of the preceding claims, wherein the membranes of the two contactors are polymeric or inorganic or comprising both polymeric materials and inorganic materials.

11. The process according to the preceding claim, wherein the membranes are modules of the tubular type, capillary fibers, hollow fibers, flat modules or spiral modules.

12. Use of the process described in claims 1-11 in the field of medicine.

13. Use of the process according to the preceding claim, in medical interventions in which the subject is anesthetized.

## Amended claims

Statement under Art. 19.1 PCT
**Novelty (Art. 33(2) PCT) and inventive step (Art. 33(3) PCT)**

In view of the examiner's novelty and inventive step objections considering D1, D2 and D3, the applicant amended claim 1 and introduce further distinguishing characteristics not anticipated or suggested in any of the cited documents, namely regarding the absorption and stripping of carbon dioxide steps, in the described process.

Since the process describe in claim 1 is new and inventive, new claim 10 is also new and inventive.

The dependent claims are used to define preferred embodiments of the independent claims, and therefore they are also new and inventive. Indeed, according to the Guidelines C-IV 11.13, it is recognized that if the independent claim is new and inventive, no need to investigate the novelty and inventive step of the dependent claims

**Clarity (Art 6 PCT)**

In view of the examiner's clarity objections a new set of amended claims is being filed in order to overcome the raised clarity issues.

Further to the present amendments the applicant would like address the examiner's observation regarding the term "room temperature" in original claim 8. In this context the applicant presents that it is understood to one skilled in the art, that "room temperature" is the temperature in which the person is accustomed to working comfortably, ranging approximately between 15 to 30 °C, preferably 20 to 25 °C, more preferably between 21 at 23 °C, however without restricting temperatures above or below these limits and provided acceptable and recognized as ambient or "room temperature", i.e. indoor.
